# FASCICULE DE BREVET EUROPEEN

(11) **EP 1 524 543 B1**
(45) Date de publication et mention de la délivrance du brevet: **05.11.2008**
(21) Numéro de dépôt: 04292381.3
(22) Date de dépôt: 06.10.2004
(51) Int. Cl.: G02B 23/24, A61B 1/00

(54) **Endoscope à éclairage ultraviolet**
Endoskop mit ultravioletter Bestrahlung
Endoscope with ultraviolet illumination

(30) Priorité: 16.10.2003 FR 0312063
(43) Date de publication de la demande: 20.04.2005
(73) Titulaire: SNECMA, 75015 Paris (FR)
(72) Inventeur: Bonningue, Isabelle, 91450 Soisy sur Seine (FR); Le Quellec, John, 77000 Vaux le Penil (FR); Rovegno, Jean, 13600 La Ciotat (FR)
(74) Mandataire: Ramey, Daniel

(56) Documents cités:
- EP-A- 0 350 603
- DE-A- 2 917 436
- DE-A- 19 512 518
- GB-A- 895 893
- GB-A- 2 025 655
- US-A- 3 880 148
- US-A- 4 195 904
- US-A- 4 727 859
- US-B1- 6 560 013
- WARREN J. SMITH: "Modern optical engineering" 1990, MCGRAWHILL INC , USA * page 171 *

## Description

L'invention concerne un endoscope à éclairage ultraviolet et à visée distale déviée destiné notamment à l'observation de défauts présentés par des pièces mécaniques et mis en évidence au moyen de produits de ressuage.

L'invention s'applique à l'endoscopie à vocation industrielle, notamment dans le domaine aéronautique, pour l'inspection visuelle de pièces internes difficilement accessibles, telles par exemple que des aubes de rotor de turboréacteur ou de turbopropulseur, au moyen d'un endoscope que l'on fait passer par un orifice de faible diamètre (par exemple de 9 mm) qui est ménagé à cet effet dans le carter de la machine à contrôler, cette technique présentant l'avantage de ne pas nécessiter un démontage préalable, total ou partiel de cette machine.

On connaît des endoscopes qui sont constitués essentiellement d'une sonde rigide destinée à être introduite dans une cavité obscure et équipée de moyens d'éclairage d'un objet à inspecter et de moyens optiques fournissant à l'utilisateur une image de cet objet. Ces moyens optiques comprennent en général un objectif distal de formation d'image, et des moyens de transmission d'image formés d'une série de lentilles et d'une lentille oculaire proximale dont le déplacement longitudinal permet un réglage de la netteté de l'image observée par l'utilisateur. Ces moyens optiques sont de préférence conçus de façon à ce que l'image transmise par la lentille oculaire ne soit pas inversée par rapport à la réalité. Les moyens d'éclairage comprennent en général un faisceau de fibres optiques dont l'extrémité distale est disposée à proximité de l'objectif distal pour éclairer l'objet, le faisceau de fibres étant relié à son extrémité proximale à une source de lumière.

Il existe notamment des endoscopes à visée axiale dans lesquels l'axe optique de l'objectif distal est confondu avec l'axe longitudinal de l'endoscope. Les moyens d'éclairage de ces endoscopes sont constitués d'un faisceau de fibres optiques dont l'extrémité distale forme en général une couronne d'éclairage autour de l'objectif distal.

On connaît également des endoscopes à visée déviée, dans lesquels l'axe de la visée optique est incliné par rapport à l'axe longitudinal de l'endoscope. Les moyens optiques d'observation d'un tel endoscope comprennent un prisme déviateur distal qui est en général un prisme à réflexion unidirectionnelle de l'image, un tel prisme étant en général appelé "prisme à réflexion partielle". Dans ce cas, les moyens de transmission d'image montés dans l'endoscope comprennent en général un prisme correcteur proximal qui redresse l'image inversée fournie par le prisme déviateur distal.

Les moyens d'éclairage d'un endoscope à visée distale déviée sont en général constitués d'un faisceau de fibres optiques dont l'extrémité distale est coudée pour constituer une fenêtre d'éclairage latérale entre le prisme déviateur distal et l'extrémité distale de l'endoscope, l'axe d'éclairage étant sensiblement parallèle à l'axe de visée.

Les fibres optiques d'éclairage sont des fibres de verre capables de transmettre sans affaiblissement sensible les composantes spectrales d'une lumière blanche fournie par une source de lumière du type lampe quartz-iode ou lampe xénon. Ces moyens d'éclairage sont inadaptés à la transmission d'une lumière ultraviolette produite par exemple par une lampe à vapeur de mercure, et il faut pour transmettre le rayonnement ultraviolet utiliser des fibres de quartz ou de matière plastique appropriée, ou encore un conducteur liquide (une gaine remplie d'un liquide approprié transparent au rayonnement ultraviolet), ces conducteurs de lumière ayant une certaine rigidité qui ne permet pas de couder leur extrémité distale avec un rayon de courbure suffisamment faible pour permettre leur montage dans un endoscope.

On a résolu ce problème dans la technique antérieure en associant à un endoscope à visée axiale comprenant un conducteur de lumière ultraviolette, un embout distal comportant un miroir de renvoi, mais cette solution est peu satisfaisante en raison de la limitation du champ optique et de la dégradation dans le temps du rendement de la réflexion de la lumière ultraviolette par le miroir, les défauts et salissures du miroir créant des pertes énergétiques qui ne permettent pas un éclairage d'un objet avec une intensité suffisante et qui nuisent à l'observation en renvoyant une image diffuse de l'objet éclairé.

Le document US-B1-6,560,013 décrit un endoscope comprenant à son extrémité distale une pluralité de prismes d'éclairage et d'observation dont certains sont pivotants autour d'un axe perpendiculaire à l'axe de l'endoscope, pour modifier la direction de visée.

Le document GB-A-895,893 décrit un endoscope à visée distale déviée destiné à être fixé à demeure sur un réacteur nucléaire pour en observer son centre, cet endoscope comprenant trois prismes déviateurs à son extrémité distale.

Le document US-A-3,880,148 décrit un endoscope comprenant à son extrémité distale deux prismes d'éclairage et deux prismes d'observation, certains de ces prismes étant montés rotatifs autour d'un axe perpendiculaire à l'axe de l'endoscope.

Le document DE-A1-195 12 518 décrit un endoscope pour le traitement de tumeurs dans le corps humain.

Le document EP 0 350 603 décrit un endoscope à visée distale déviée pour la détection de défauts de pièces au moyen d'une technique de ressuage en éclairage ultraviolet. Il comprend deux prismes fixes d'observation et d'éclairage.

L'invention a notamment pour but d'apporter une solution simple, efficace et peu coûteuse aux problèmes de la technique antérieure.

Elle a pour objet un endoscope à éclairage ultraviolet et à visée distale déviée, qui ne présente pas les inconvénients précités.

Elle propose à cet effet un endoscope à visée distale déviée, tel que défini dans la revendication 1.

La disposition transversale côte à côte des deux prismes est peu encombrante et facilite l'utilisation de l'endoscope.

Les moyens de guidage de lumière sont des moyens de guidage de lumière ultraviolette et débouchent longitudinalement à l'extrémité distale du tube sur le prisme déviateur des moyens d'éclairage.

Cela permet d'utiliser des moyens de guidage de lumière qui ne sont pas coudés.

Avantageusement, l'endoscope comprend des moyens de commande du pivotement de la platine depuis l'extrémité proximale du tube.

Cela permet d'avoir une visée prograde, latérale ou rétrograde et d'améliorer les conditions d'observation des objets à inspecter.

L'écran opaque est monté entre les deux prismes, pour éviter qu'une lumière d'éclairage parasite soit captée directement par les moyens d'observation, cet écran opaque étant par exemple fixé sur ou formé par la platine portant les deux prismes.

On peut prévoir dans l'endoscope selon l'invention que l'axe de visée et l'axe d'éclairage sont sensiblement convergents à une distance latérale déterminée du tube de l'endoscope, pour un meilleur éclairage et une meilleure observation des objets situés à cette distance.

L'invention sera mieux comprise et d'autres caractéristiques, détails et avantages de celle-ci apparaîtront plus clairement à la lecture de la description qui suit, faite à titre d'exemple en référence aux dessins annexés dans lesquels :
- la figure 1 est une vue schématique en perspective d'un endoscope de la technique antérieure ;
- la figure 2 est une vue schématique en perspective d'un endoscope selon l'invention ;
- la figure 3 est une vue partielle illustrant un montage particulier des prismes de l'endoscope selon l'invention ;
- la figure 4 est une autre vue schématique éclatée en perspective représentant le montage pivotant des prismes de l'endoscope selon l'invention.

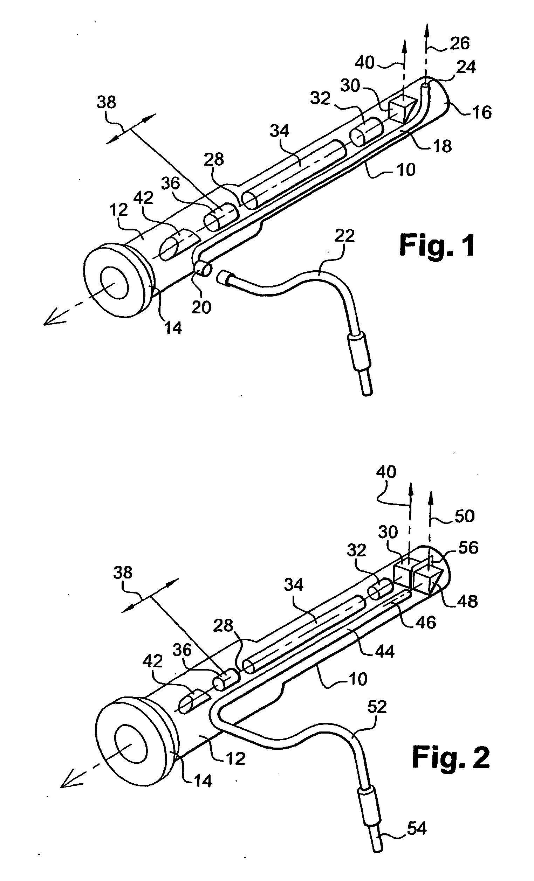

On se réfère d'abord à la figure 1 qui représente un endoscope connu, comprenant essentiellement un tube rectiligne rigide 10 contenant des moyens d'éclairage et des moyens d'observation, ce tube 10 comportant une extrémité proximale formant une poignée 12 et munie d'une bonnette 14, et une extrémité distale 16 destinée à être introduite à l'intérieur d'un carter, par exemple, par un orifice de faible diamètre de celui-ci.

Les moyens d'éclairage logés dans le tube 10 comprennent un conducteur de lumière 18, tel qu'un câble de fibres optiques, dont l'extrémité proximale est montée dans une embase de connexion 20 portée par la poignée 12 de l'endoscope et destinée à recevoir l'extrémité d'un câble d'éclairage 22 relié à une source d'éclairage en lumière blanche. L'extrémité distale 24 du conducteur de lumière 18 est coudée, par exemple sensiblement à angle droit, et détermine un axe d'éclairage 26 qui est incliné par rapport à l'axe longitudinal 28 de l'endoscope.

Les moyens d'observation comprennent un prisme de visée 30 qui est monté sur l'axe longitudinal 28 de l'endoscope à l'extrémité distale de celui-ci et qui est associé à des moyens de transmission d'images qui sont logés dans le tube 10 sur l'axe 28 et comportent un objectif 32 de formation d'image et une série de lentilles achromatiques 34, ainsi qu'une lentille oculaire 36 mobile axialement et dont le déplacement longitudinal 38 permet un réglage de la mise au point de l'image. Le prisme 30 détermine un axe de visée 40 qui est sensiblement parallèle à l'axe d'éclairage 26 et est par exemple un prisme à réflexion unidirectionnelle produisant une image inversée. Dans ce cas, un prisme correcteur 42 peut être monté sur l'axe 28 de l'endoscope entre la lentille 36 et la bonnette 14, pour redresser l'image.

Cet endoscope connu permet un éclairage en lumière blanche des objets à inspecter, mais ne peut être utilisé pour la détection de défauts mis en évidence par une technique de ressuage, qui nécessite l'éclairage de ces défauts en lumière ultraviolette.

On se réfère maintenant à la figure 2, qui représente schématiquement un mode de réalisation d'un endoscope selon l'invention.

Cet endoscope comprend le même tube rigide rectiligne 10 que celui de la figure 1 et des moyens d'observation qui sont semblables à ceux déjà décrits et qui comprennent un prisme de visée 30 dont l'axe de visée 40 est incliné par rapport à l'axe longitudinal 28 du tube 10, un objectif 32, une série de lentilles achromatiques 34, une lentille oculaire 36 déplaçable axialement comme indiqué en 38 pour le réglage de la netteté de l'image, un prisme redresseur 42 et une bonnette 14 montée à l'extrémité de la poignée 12 de l'endoscope.

Les moyens d'éclairage comprennent des moyens 44 de guidage d'une lumière ultraviolette, qui sont contenus dans le tube 10 et s'étendent le long de l'axe 28, l'extrémité distale 46 de ces moyens de guidage étant rectiligne et parallèle à l'axe 28 pour déboucher sur un prisme 48 à réflexion unidirectionnelle, du même type que le prisme 30 des moyens d'observation, le prisme 48 déviant le faisceau lumineux ultraviolet sortant des moyens de guidage 44 pour diriger ce faisceau dans une direction 50 qui est sensiblement parallèle à l'axe de visée 40.

L'extrémité proximale 52 des moyens 44 de guidage de lumière est extérieure à l'endoscope et comporte un embout 54 de connexion à une source de lumière ultraviolette.

Dans ce cas, les moyens 44 de guidage de lumière ultraviolette peuvent être des fibres de quartz, des fibres de matière plastique appropriée ou un conducteur liquide (une gaine remplie d'un liquide approprié transparent au rayonnement ultraviolet).

En variante, l'extrémité proximale des moyens 44 de guidage de lumière ultraviolette peut être reliée à une embase de connexion portée par la poignée 12 de l'endoscope, comme dans le mode de réalisation de la figure 1, et un câble conducteur de lumière ultraviolette muni d'un embout approprié est raccordable à cette embase. Les moyens de guidage de lumière 44 sont de préférence des fibres de quartz et le câble externe est de préférence un câble liquide.

Le prisme d'éclairage 48 est en quartz ou en verre transparent au rayonnement ultraviolet et transmet avec un très bon rendement (par exemple supérieur à 99 %) la lumière ultraviolette fournie par la source externe.

Dans tous les cas, la source de lumière ultraviolette reliée aux moyens de guidage 44 comporte un filtre passe-bande centré sur la longueur d'onde ultraviolette retenue par l'utilisateur de l'endoscope.

Les prismes 40 et 48 sont montés au voisinage immédiat de l'extrémité distale du tube 10, aussi près que possible de cette extrémité, et un écran 56 opaque au rayonnement ultraviolet est monté entre les deux prismes, pour éviter toute entrée parasite de la lumière d'éclairage dans le prisme 30, l'objectif 32 et les lentilles 34 des moyens d'observation.

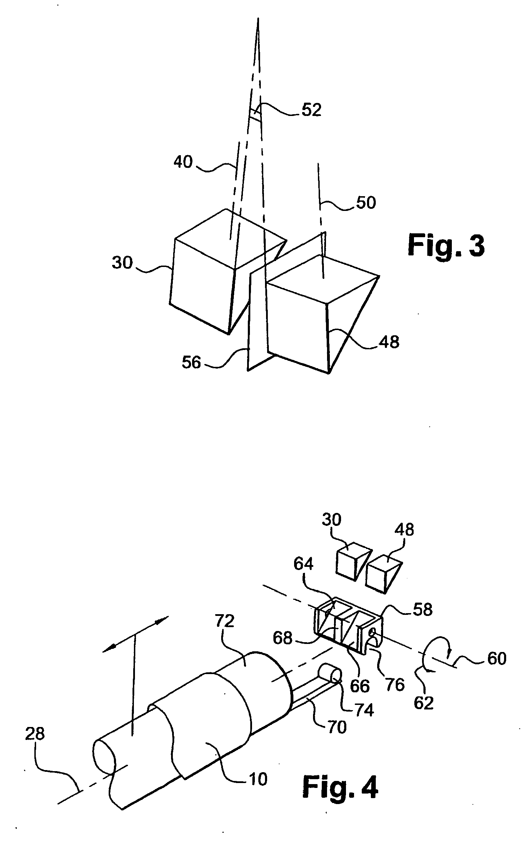

Comme représenté schématiquement en figure 3, au moins l'un des prismes 30, 48 est incliné latéralement en direction de l'autre prisme, de façon à ce que les axes de visée 40 et d'éclairage 50 convergent, au moins approximativement, à une certaine distance de l'endoscope, en faisant entre eux un angle faible 52. On améliore ainsi l'éclairage et l'observation des objets qui sont situés à une distance radiale de l'endoscope correspondant approximativement à la distance à laquelle les axes 40 et 50 convergent.

Dans une variante non couverte par la revendication 1, les orientations de ces axes par rapport à l'axe longitudinal 28 de l'endoscope peuvent être fixes et prédéterminées, avec une visée prograde (orientée vers l'avant de l'endoscope), latérale (à 90 ° environ de l'axe longitudinal 28) ou rétrograde (orientée vers l'arrière de l'endoscope).

Selon l'invention et comme représenté schématiquement en figure 4, les prismes 30 et 48 sont montés sur une même platine 58 qui est elle-même montée pivotante autour d'un axe transversal 60 comme indiqué par la double flèche 62, l'axe transversal 60 étant perpendiculaire à l'axe longitudinal 28 de l'endoscope et perpendiculaire ou sensiblement perpendiculaire aux axes de visée 40 et d'éclairage 50, ces axes étant parallèles ou bien convergents comme représenté en figure 3.

La platine 58 peut comprendre deux logements 64, 66 destinés respectivement à recevoir le prisme 30 et le prisme 48, ces logements étant séparés par une cloison intermédiaire 68 formant écran entre les deux prismes.

L'axe 60 de pivotement de la platine 58 est matérialisé par tous moyens appropriés, par exemple par deux doigts diamétralement opposés portés par l'extrémité distale du tube 10 et s'engageant dans deux orifices de la platine.

Avantagement, des moyens sont prévus pour faire pivoter la platine 58 autour de l'axe 60 depuis l'extrémité proximale de l'endoscope, ces moyens comprenant par exemple une patte longitudinale 70 dont l'extrémité distale est montée articulée sur la platine 58 autour d'un axe parallèle à l'axe 60, et dont l'extrémité proximale est solidaire d'un tube 72 monté coulissant à l'intérieur du tube 10 de l'endoscope, l'extrémité proximale de ce tube 72 étant déplaçable par l'utilisateur en translation axiale.

Par exemple, l'extrémité distale de la patte 70 comporte un doigt cylindrique transversal 74 reçu dans un évidement semi-cylindrique transversal 76 de la partie inférieure de la platine 58.

Cela permet à l'utilisateur de commander la rotation de la platine 58 autour de l'axe 60, et donc l'orientation des axes de visée 40 et d'éclairage 50 autour de cet axe, pour une visée prograde, latérale ou rétrograde.

## Revendications

1. Endoscope à visée distale déviée pour la détection de défauts de pièces au moyen d'une technique de ressuage, comprenant un tube rigide (10) contenant des moyens d'éclairage et des moyens d'observation,
les moyens d'observation comprenant un prisme de visée (30) monté à l'extrémité distale du tube et définissant un axe latéral de visée (40), et des moyens de transmission d'image s'étendant entre le prisme de visée (30) et l'extrémité proximale du tube,
les moyens d'éclairage comprenant un prisme d'éclairage (48) monté à l'extrémité distale du tube et définissant un axe d'éclairage (50) sensiblement parallèle à l'axe de visée (40), **caractérisé en ce que :**
- les moyens d'éclairage comprennent des moyens (44) de guidage de la lumière qui sont transparents au rayonnement ultraviolet et qui s'étendent le long de l'axe du tube pour déboucher sur le prisme d'éclairage (48), qui dévie le faisceau lumineux ultraviolet sortant des moyens de guidage (44) pour le diriger dans une direction sensiblement parallèle à l'axe de visée (40), ce prisme étant à réflexion unidirectionnelle et étant réalisé en quartz ou en verre transparent au rayonnement ultraviolet,
- et le prisme de visée (30) et le prisme d'éclairage (48) sont montés transversalement côte à côte à l'extrémité distale du tube sur une même platine (58) qui peut pivoter autour d'un axe transversal (60) perpendiculaire ou sensiblement perpendiculaire aux axes de visée (40) et d'éclairage (50), et sont séparés par un écran (56) opaque au rayonnement ultraviolet qui est porté ou formé par la platine (58) portant les prismes.

2. Endoscope selon la revendication 1, **caractérisé en ce que** les moyens de transmission d'image comprennent un objectif (32) et une série de lentilles achromatiques (34) ainsi qu'un prisme correcteur proximal (42) de redressement de l'image inversée fournie par le prisme de visée (30).

3. Endoscope selon la revendication 1, **caractérisé en ce qu'**il comprend des moyens (70, 72, 74) de commande du pivotement de la platine (58) depuis l'extrémité proximale du tube (10).

4. Endoscope selon l'une des revendications précédentes, **caractérisé en ce que** l'axe de visée (40) et l'axe d'éclairage (50) sont sensiblement convergents.

5. Endoscope selon l'une des revendications précédentes, **caractérisé en ce que** les moyens (44) de guidage de lumière ultraviolette comprennent un câble de fibres de quartz, ou un câble de fibres de matière plastique appropriée ou une gaine étanche remplie d'un liquide approprié.

6. Endoscope selon l'une des revendications précédentes, **caractérisé en ce que** les moyens (44) de guidage de la lumière ultraviolette comprennent une extrémité proximale (52) externe au tube (10) et munie d'un embout (54) de connexion à une source de lumière ultraviolette.

7. Endoscope selon l'une des revendications 1 à 5, **caractérisé en ce que** les moyens (44) de guidage de lumière ultraviolette ont une extrémité proximale montée dans une embase solidaire du tube (10) et destinée à être reliée à un conducteur de lumière extérieur au tube (10).

8. Endoscope selon la revendication 7, **caractérisé en ce que** le conducteur de lumière extérieur au tube (10) est une gaine remplie d'un liquide approprié.

## Claims

1. An endoscope with deflected distal viewing for detecting parts defects by means of penetration test substances, comprising a rigid tube (10) containing lighting means and observation means,
the observation means comprising a viewing prism (30) mounted at the distal end of the tube and defining a lateral viewing axis (40), and image transmission means extending between the viewing prism (30) and the proximal end of the tube,
the lighting means comprising a lighting prism (48) mounted at the distal end of the tube and defining a lighting axis (50) substantially parallel to the viewing axis (40), **characterized in that:**
- the lighting means comprise light guiding means (44) that are transparent to ultraviolet radiation and that extend along the tube axis for opening out onto the lighting prism (48), which deflects the ultraviolet light beam exiting from the guiding means (44) for orienting it in a direction substantially parallel to the viewing axis (40), this prism being an unidirectional reflection prism and being made of quartz or of glass transparent to the ultraviolet radiation,
- and the viewing axis (30) and the lighting axis (48) are mounted transversely side by side at the distal end of the tube on a common cradle (58) capable of pivoting about a transverse axis (60) perpendicular or substantially perpendicular to the viewing axes (40, 50), and are separated by a screen (56) opaque to the ultraviolet radiation that is carried or formed by the cradle (58) carrying the prisms.

2. An endoscope according to claim 1, **characterized in that** the image transmission means comprise an objective lens (32) and a series of achromatic lenses (34) together with a proximal correcting prism (42) for rectifying the inverted image supplied by the viewing prism (30).

3. An endoscope according to claim 1, **characterized in that** it includes means (70, 72, 74) for controlling pivoting of the cradle (58) from the proximal end of the tube (10).

4. An endoscope according to any preceding claim, **characterized in that** the viewing axis (40) and the lighting axis (50) are substantially convergent.

5. An endoscope according to any preceding claim, **characterized in that** the ultraviolet light guide means (44) comprise a cable of quartz fibers, or a cable of fibers made of an appropriate plastics material, or a leakproof sheath filled with an appropriate liquid.

6. An endoscope according to any preceding claim, **characterized in that** the ultraviolet light guide means (44) include a proximal end (52) outside the tube (10) and provided with an endpiece (54) for connection to an ultraviolet light source.

7. An endoscope according to any one of claims 1 to 5, **characterized in that** the ultraviolet light guide means (44) have a proximal end mounted in a socket secured to the tube (10) and suitable for being connected to a light conductor external to tube (10).

8. An endoscope according to claim 7, **characterized in that** the light conductor external to the tube (10) is a sheath filled with an appropriate liquid.

## Patentansprüche

1. Endoskop mit abgelenkter Fernoptik zur Erkennung von Fehlern an Werkstücken mittels einer Ausschwitz-Technik, bestehend aus einem starren Rohr (10), das Beleuchtungsmittel und Betrachtungsmittel enthält, wobei die Betrachtungsmittel ein Optikprisma (30), das am distalen Ende des Rohrs angebracht ist und eine zur Seite gerichtete Optikachse (40) bildet, und Bildübertragungsmittel, die sich zwischen dem Optikprisma (30) und dem proximalen Ende des Rohrs erstrecken, umfassen, wobei die Beleuchtungsmittel ein Beleuchtungsprisma (48) umfassen, das am distalen Ende des Rohrs angebracht ist und eine im Wesentlichen parallel zur Optikachse (40) verlaufende Beleuchtungsachse (50) bildet,
**dadurch gekennzeichnet,**
**dass**
- die Beleuchtungsmittel Mittel (44) zum Führen des Lichts umfassen, die für Ultraviolettstrahlung transparent sind und sich entlang der Achse des Rohrs erstrecken, um auf das Beleuchtungsprisma (48) zu treffen, das den aus den Führungsmitteln (44) austretenden Ultraviolettlichtstrahl ablenkt, um ihn in einer im Wesentlichen parallel zur Optikachse (40) verlaufenden Richtung zu lenken, wobei dieses Prisma ein Prisma mit einseitig gerichteter Reflexion ist und aus für Ultraviolettstrahlung transparentem Quarz oder Glas ausgeführt ist,
- und das Optikprisma (30) und das Beleuchtungsprisma (48) in Querrichtung nebeneinander am distalen Ende des Rohrs auf einer und derselben Platte (58) angebracht sind, welche um eine Querachse (60) schwenken kann, die im rechten Winkel oder im Wesentlichen im rechten Winkel zu der Optikachse (40) und der Beleuchtungsachse (50) verläuft, und durch einen für Ultraviolettstrahlung undurchlässigen Schirm (56) voneinander getrennt sind, welcher von der Platte (58) gebildet oder gehalten wird, an der die Prismen sitzen.

2. Endoskop nach Anspruch 1,
**dadurch gekennzeichnet,**
**dass** die Bildübertragungsmittel ein Objektiv (32) und eine Reihe von farblosen Linsen (34) sowie ein Nahkorrekturprisma (42) zur Umkehr des von dem Optikprisma (30) abgegebenen umgekehrten Bildes umfassen.

3. Endoskop nach Anspruch 1,
**dadurch gekennzeichnet,**
**dass** es Mittel (70, 72, 74) zur Steuerung der Schwenkbewegung der Platte (58) vom proximalen Ende des Rohrs (10) aus umfasst.

4. Endoskop nach einem der vorherigen Ansprüche,
**dadurch gekennzeichnet,**
**dass** die Optikachse (40) und die Beleuchtungsachse (50) im Wesentlichen konvergierend verlaufen.

5. Endoskop nach einem der vorherigen Ansprüche,
**dadurch gekennzeichnet,**
**dass** die Mittel (44) zum Führen von ultraviolettem Licht ein Quarzfaserkabel oder ein Faserkabel aus geeignetem Kunststoff oder eine dichte Hülle, die mit einer geeigneten Flüssigkeit gefüllt ist, umfassen.

6. Endoskop nach einem der vorherigen Ansprüche,
**dadurch gekennzeichnet,**
**dass** die Mittel (44) zum Führen des ultravioletten Lichts ein proximales Ende (52), das sich außerhalb des Rohrs (10) befindet und mit einem Endstück (54) zum Anschließen an eine UV-Lichtquelle versehen ist.

7. Endoskop nach einem der Ansprüche 1 bis 5,
**dadurch gekennzeichnet,**
**dass** die Mittel (44) zum Führen des ultravioletten Lichts ein proximales Ende aufweisen, das in einem mit dem Rohr (10) fest verbundenen Sockel sitzt, der dazu bestimmt ist, mit einem außerhalb des Rohrs (10) befindlichen Lichtleiter verbunden zu werden.

8. Endoskop nach Anspruch 7,
**dadurch gekennzeichnet,**
**dass** der außerhalb des Rohrs (10) befindliche Lichtleiter eine mit einer geeigneten Flüssigkeit gefüllte Hülle ist.
